Europäisches Patentamt

European Patent Office

Office européen des brevets

(11) Publication number: **0 209 204**
A1

# EUROPEAN PATENT APPLICATION

(21) Application number: 86201252.3

(22) Date of filing: 16.07.86

(51) Int. Cl.⁴: **C 12 N 15/00**, C 12 N 1/20
// (C12N1/20, C12R1:19)

(30) Priority: 17.07.85 NL 8502061

(43) Date of publication of application: 21.01.87
Bulletin 87/4

(84) Designated Contracting States: BE DE FR GB LU NL

(71) Applicant: **Stichting Katholieke Universiteit,**
**Toernooiveld 1, NL-6525 ED Nijmegen (NL)**

(72) Inventor: **Peeters, Bernardus Petrus Hubertus,**
**Zwanebloemweg 13, NL-9753 JE Haren (NL)**
Inventor: **Konings, Rudolph Nicolaas Hendrik,**
**Kouwenberg 36, NL-5431 GZ Cuyk (NL)**
Inventor: **Schoenmakers, Johannes Gerardus Ghislain,**
**Knollenberg 18, NL-6585 WK Mook (NL)**

(74) Representative: **Smulders, Theodorus A.H.J. et al,**
**Vereenigde Octrooibureaux Nieuwe Parklaan 107,**
**NL-2587 BP 's-Gravenhage (NL)**

(54) New vector plasmids, their construction and use, and microorganisms in which they are incorporated.

(57) This invention relates to new vector pasmids which, in a starting vector, such as a pUC plasmid, contain, in opposite orientation relative to each other

(a) the replication origin + morphogenetic signal of a filamentous E.coli bacteriophage having F pili, and

(b) the replication origin + morphogenetic signal of a filamentous E. coli bacteriophage having N pili. By means of these vector plasmids each of the two strands of a DNA insertion can be selectively packaged in single-stranded DNA containing phagelike particles.

Title: new vector plasmids, their construction and use, and microorganisms in which they are incorporated.

This invention relates to new vector pladmids for use in recombinant DNA techniques, and to a method for the construction of said plasmids and their use, as well as to microorganisms, in particular E. coli, in which they are incorporated.

During the last few years a large number of recombinant DNA molecules have been constructed in which several unique biological functions have been combined to yield attractive tools for cloning and manipulating DNA (Old & Primrose, 1982). These vectors are generally characterized by the presence of a genetic determinant the phenotype of which is altered by insertional inactivation. In addition these vectors contain multiple unique restriction enzyme cleavage sites allowing the direct cloning of a variety of restriction fragments.

More recently cloning vehicles have been constructed which allow the direct identification of recombinant clones without the need of replica plating. The most versatile of these vectors are the filamentous phage vectors and pUC plasmids which make use of complementation of the lacZ mutation of suitable host strains (Vieira & Messing, 1982; Messing, 1983). Dente et al (1983) have described the construction of a family of cloning vectors in which the properties of the pUC plasmids and the M13mp phages are combined. By cloning a fragment containing the functional replication origin and the morphogenetic signal of the Ff phage f1 into the pUC plasmids, new plasmid vectors (pEMBL plasmids) were obtained. In the absence of helper phages replication of these plasmids is dependent on the ColE1 origin.

Upon infection of cells harboring these plasmids with phage IRl (a derivative of fl), the plasmids enter the Ff mode of replication leading to the selective packaging of one strand (F strand) into phage-like particles.

Although the M13mp phages and the pEMBL plasmids are particularly suited for the preparation of large amounts of single-stranded DNA which, for instance, can be used for DNA sequence analysis, they suffer from one great drawback. With the aid of these vectors only one of the DNA strands, i.e. the strand on which the origin of viral strand replication is located, can be packaged. Since it is generally necessary to establish the nucleotide sequence of both strands of a DNA fragment, the fragment of interest must therefore be cloned in both orientations in the same vector. Frequently, the sequence of a relatively large DNA molecule is established via shotgun cloning of randomly generated fragments (Sanger et al., 1980; Messing et al., 1981; Peeters et al., 1985). In this case sequencing of both DNA strands requires the presence in the DNA library of clones containing overlapping fragments whose orientation is reversed. If this requirement is not met, the fragment of interest must be recovered from the particular recombinant plasmid and recloned in the opposite orientation. Alternatively the nucleotide sequence can be established via sequence analysis using double-stranded DNA, either directly or after exonuclease III treatment (Wallace et al., 1981; Guo & Hu, 1982). It is obvious that either method requires the isolation and purification of intracellular plasmid DNA, a process which is rather time-consuming, particularly when a large number of clones are involved. Moreover the results of sequence analysis using double-stranded DNA are not routinely as good as those using a single-stranded DNA template (Deininger, 1983).

The present invention provides a new vector plasmid which overcomes these drawbacks in that it is built up from a starting vector incorporating, in opposite orientation relative to each other:

a.) the replication origin + morphogenetic signal, or an active mutant thereof, of a filamentous bacteriophage of E. coli bacteria having F pili; and

b) the replication origin + morphogenetic signal, or an active mutant thereof, of a filamentous bacteriophage of E. coli bacteria having N pili.

More specifically the invention relates to such a vector plasmid which contains:

a) the replication origin + morphogenetic signal, or an active mutant thereof, of the filamentous bacteriophage Ff(M13, fd and f1) or a mutant thereof, and

b) the replication origin + morphogenetic signal, or an active mutant thereof, of the filamentous bacteriophage IKe or a mutant thereof.

Preferably, the new vector plasmid is based on a pUC plasmid as the starting vector, more preferably based on the plasmid pUC9 as the starting vector.

A particular embodiment of such a new vector plasmid is based on pUC9, in which (a) has been inserted in the unique recognition sequence for the restriction enzyme NarI and (b) has been inserted in the unique recognition sequence for the restriction enzyme NdeI, or the other way round.

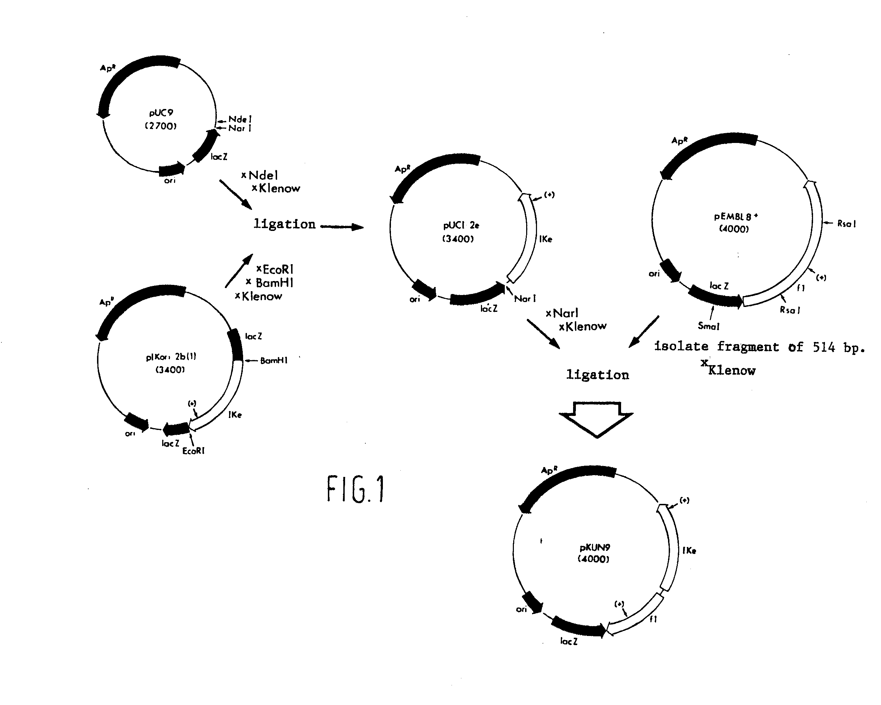

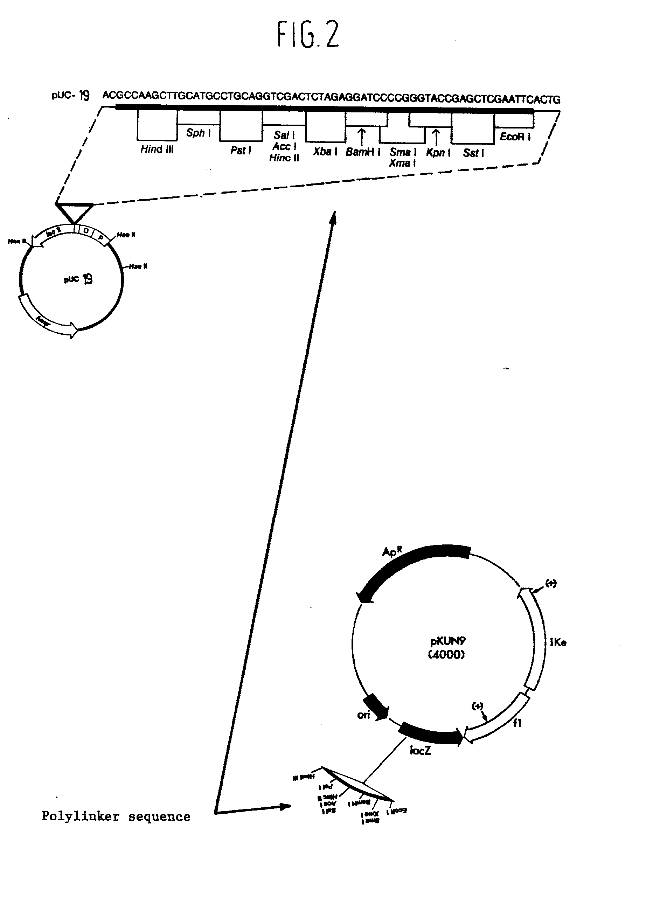

In this specification an example of such a plasmid is given, namely, plasmid pKUN9 having the structure shown in Fig. 1. Another example is the pKUN9-derived plasmid pKUN19 having the structure illustrated in Fig. 2.

The invention also comprises vector plasmids as defined above, into which a piece of double-stranded DNA has been inserted at a suitable location.

The invention also extends to microorganisms, in particular E. coli bacteria, containing a vector plasmid according to the invention. Preferably the vector plasmid is incorporated into E. coli bacteria sensitive to infection by F-specific and/or N-specific filamentous bacteriophages i.e., E. coli bacteria having F pili and/or N pili, owing to the presence of Ftra and/or Ntra genes.

The invention further extends to the use of the new vector plasmids according to the invention and to methods of constructing the new vector plasmids according to the invention.

Thus the invention provides a method of selectively packaging one of the strands or each of the two strands of a piece of double-stranded DNA in single-stranded DNA containing phage-like particles, in which E. coli bacteria susceptible to infection by F-specific filamentous bacteriophages and containing a vector plasmid according to the invention in which the piece of double-stranded DNA has been inserted are infected with F-specific filamentous bacteriophages, and after incubation the phage-like transducing particles are isolated, and/or E. coli bacteria susceptible to infection by N-specific filamentous bacteriophages and containing a vector plasmid according to the invention are infected with N-specific filamentous bacteriophages, and after incubation the phage-like transducing particles are isolated, or a portion of a population of E. coli bacteria sensitive to both F-specific and N-specific filamentous bacteriophages and containing a vector plasmid according to the invention are infected with F-specific filamentous bacteriophages and another part of said population with N-specific filamentous bacteriophages, and after incubation the phage-like transducing particles formed are isolated.

The invention extends to the sequencing of each of the stands of the piece of DNA concerned, thus separately obtained, and more generally to the use of a vector plasmid according to the invention, in which a piece of double-stranded DNA has been inserted, for cloning DNA or messenger RNA, DNA sequencing, site directed mutagenesis , S1 mapping, expressing cloned DNA, and the preparation of a single-stranded hybridization probe.

Broadly, the new vector plasmids according to the invention are constructed by cloning (a) or (b) in a restriction enzyme recognition sequence of a parental vector, selecting transformants, cloning (b) or (a) therein in another restriction enzyme recognition sequence of the parental vector, and selecting transformants.

More specifically the procedure comprises cloning (a) or (b) in the unique recognition sequence for the restriction enzyme NarI or that for the restriction enzyme NdeI of pUC9, selecting transformants by means of a polA mutant of E. coli infected with F-specific or N-specific filamentous bacteriophages, subsequently cloning (b) or (a) in one of the resulting recombinant plasmids in the unique recognition sequence not yet used, and selecting transformants by means of a polA mutant of E. coli infected with N-specific or F-specific filamentous bacteriophages.

A preferred embodiment of the invention, which is described in more detail in the experimental section, is a method of constructing the vector plasmid pKUN9, which comprises treating the plasmid pUC9 with the restriction enzyme NdeI and Klenow DNA polymerase I to form a blunt-ended, linear, double-stranded DNA, ligating said linear DNA with a blunt-ended, linear, double-stranded DNA produced by treating the plasmid pIKori 2b (1) with the restriction enzymes EcoRI and BamHI, isolating the fragment containing the (+)origin + morphogenetic signal of IKe, and treating it with Klenow DNA polymerase I, selecting transformants containing the replication origin + morphogenetic signal of IKe inserted in the NdeI position of pUC9 by transformation of a polA mutant of E. coli infected with IKe or a mutant thereof, and selection of ampicillin -resistant colonies, isolating the recombinant plasmid containing the (+)-origin of IKe in the same orientation as the lacZ gene of pUC9, treating this plasmid with the restriction enzyme NarI and Klenow DNA polymerase I to form blunt-ended, linear, double-stranded DNA, ligating said linear DNA with a blunt-ended linear, double-stranded DNA produced by treating the plasmid pEMBL8 with the restriction enzyme RsaI, isolating the fragment containing the (+)origin + morphogenetic signal of f1, and treating it with Klenow DNA polymerase I, and selecting transformants containing the replication origin + morphogenetic signal of f1 inserted in the NarI position of pUC9 by the transformation of a polA mutant of E. coli infected with Ff or a mutant thereof, and the selection of of ampicillin-resistant colonies.

A further preferred embodiment of the invention, which will also be elucidated in the experimental section, is a method of constructing the vector plasmid pKUN19, derived from pKUN9, which comprises treating the plasmid pUC19 with the restriction enzymes EcoRI , HindIII and subsequently with HaeII, isolating the products of the digestion and adding these to the fragments produced by digestion of the plasmid pKUN9 with the restriction enzymes EcoRI, HindIII and PstI, adding ligase to this mixture, and transforming the DNA molecules formed to E. coli JM83, and selecting these on agar plates containing ampicillin and X-gal.

The plasmid pKUN19 derived from pKUN9 contains the polylinker sequence of the pUC19 plasmid. As a result, relative to pKUN19 it contains as many as four additional unique cleavage sites for restriction enzymes and hence cloning position in the lacZ gene.

As is well known, the replication of the single-stranded (ss) DNA genome of the N-specific (IKe) and F-specific (M13, fd and fl, here jointly designated Ff) filamentous bacteriophages occurs via a double-stranded intermediate called the replicative form (RF). In the DNA replication cycle three stages can be distinguished (for a review see Denhardt et al, (1978)). In the first stage the viral ssDNA is converted into RF, a process which is entirely dependent on host encoded functions and a (complementary strand) replication origin ((-)-origin) present in the single-stranded phage genome. In the second stage the RF is replicated many times yielding a large pool of progeny RF molecules. This process is initiated by the action of the phage encoded gene II protein which introduces a nick at a specific position (viral strand replication origin or (+)-origin) in the viral strand (Meyer et al., 1979), thereby creating a 3'-OH end which serves as a primer for replication by DNA polymerase according to a

-8-

rolling circle mechanism. After one round of replication gene II protein again cleaves the displaced viral strand at exactly the same position to separate the parental (+) strand from the newly synthesized (+) strand. Concomitantly, the resulting RF and ssDNA molecules are covalently closed yielding circular molecules (Meyer & Geider, 1982). Early in infection the newly synthesized ssDNA is converted into RF as in the first stage of replication. Late in infection, however, (stage three) the ssDNA is covered by the phage encoded ssDNA binding protein (gene V protein) which prevents its conversion into RF and thus leads to the synthesis of progeny ssDNA. The nucleoprotein complex of ssDNA and gene V protein is transported to the host cell membrane where packaging into mature phage particles takes place. For this process a specific DNA sequence (morphogenetic signal) contiguous to the (+)-origin is required (Dotto & Zinder, 1983)

Applicants have now found that the biological functions of the gene II proteins of IKe and Ff are not exchangeable. In addition they have found that, provided the orientations of the origins of viral strand replication are opposite to one another, recombinant plasmids that contain the (+)-origins of both IKe and Ff can be replicated by gene II protein of both IKe and Ff. In the presence of IKe gene II protein, only the IKe (+)-origin is used for replication whereas the (+)-origin of Ff is used in case gene II protein of Ff is present. Furthermore, the plasmids can be efficiently packaged into phage-like particles provided they also contain the phage morphogenetic signal. The function of this signal is coupled in cis to the presence of a functional phage (+)-origin on the same DNA strand (Dotto et al., 1981; Dotto & Zinder, 1983).

-9-

Thus in case in recombinant plasmids the orientations of the fragments of IKe and Ff that contain both the replication origin and the morphogenetic signal are opposite to each other, the DNA strand to be packaged into phage-like particles can be selected simply by infecting the cells harboring these plasmids with either IKe or Ff.

These particular properties were used for the construction of the versatile vector plasmids according to the invention.

The plasmids according to the invention are preferably based on a pUC9 plasmid. The use of this plasmid is preferred because it possesses several unique properties for cloning (Vieira & Messing, 1982).

Despite the introduction of the f1 and IKe sequences in pUC9, in accordance with the invention with the exception of the AccI site, all cloning sites in the lacZ DNA are unique. An additional advantage is that the relatively small size of the plasmid and its ability to propagate recombinant clones of the vector plasmid as a plasmid, permit the stable insertion and packaging of much larger DNA fragments than is possible using phages of the M13mp-series (Dente et al., 1983).

Plasmids according to the invention, such as pKUN9 and pKUN19, not only combine all unique properties of the single-stranded phage vectors and the pUC-plasmids, but, moreover, extend their use by making it possible to obtain either DNA strand of one recombinant plasmid separately in a single-stranded form. Undoubtedly, this property will be of special utility in the use of shotgun cloning for the establishment of the sequence of a relatively large DNA molecule. Since, by using the plasmids accor-·ding to the invention, twice the amount of sequence information

-10-

can be obtained from one single clone, the time needed for the collection of the desired sequence can be considerably reduced. Alternatively, by cloning relatively small fragments, the nucleotide sequence of both strands can be established simultaneously. This is especially useful when ambiguities are encountered in the sequence analysis of one strand.

The plasmids according to the invention are useful vectors for cloning and DNA sequencing (Sanger et al., 1980) as well as for site directed mutagenesis (Zoller & Smith, 1982), S1-mapping (Ciliberto et al., 1983), mRNA cloning (Heidecker & Messing, 1983), expression of cloned DNA (Slocombe et al., 1982) and for the production of a single-stranded hybridization probe (Hu & Messing, 1982). In the accompanying drawings,

Fig. 1 is a diagram illustrating the method for the construction of the plasmid according to the invention, pKUN9, starting from the plasmid pUC9;

Fig. 2 is a diagram illustrating the method for the construction of the plasmid according to the invention pKUN19 from the plasmid pKUN9;

Fig. 3 illustrates a portion of the nucleotide sequence of the lacZ gene present in the transducing particles isolated from infected cells. The sequence of the resulting single-stranded DNA after infection with IKe9 can only be determined with the master primer (upper sequence), while for the sequence determination of DNA from the particles obtained after infection with IR1 the reverse primer must be used (lower sequence).

The invention is illustrated in and by the following experimental section, which serves by way of illustration only

-11-

and should not be construed as limiting the invention in any way.

## A. MATERIALS AND METHODS

### (a) Bacteria, phages and plasmids

The bacterial strains used were Escherichia coli JM83, (080 lacZ DM15, ara, Dlacpro, strA, thi) (Messing, 1979), N4156 and N4156/F' (polA, end, thy, gyrA) Gellert et al., 1977). To make JM83 susceptible to infection by IKe and Ff, the compatible conjugative plasmids N3 and F'tslac: Tn5 were introduced into this strain via conjugation (Dennison & Baumberg, 1975).

Bacteriophage IR1, a derivative of f1, was used for the packaging of the F strand of plasmid pKUN9 because IR1 is resistant to negative interference by plasmids that carry a functional f1 origin (Dotto et al., 1981). Bacteriophage IKe-9 is a variant of wt IKe (Kathoon et al., 1972; Peeters et al., 1985) and was used for the packaging of the I strand of plasmid pKUN9 because infection with this phage results in a much higher yield of transducing particles than infection with wt IKe.

Plasmid pIKori-2b(1) contains an IKe DNA fragment on which both the replication origin of IKe as well as the morphogenetic

-12-

(b) Enzymes and biochemicals

All restriction enzymes, T4-DNA ligase and DNA polymerase I (Klenow fragment) were obtained from Bethesda Research Laboratories, New England Biolabs or Boehringer-Mannheim and used with the three-buffer system described by Maniatis et al.(1982) or with the buffer suggested by the supplier.

To create blunt ends, 1 $\mu$g of DNA was treated with 1 unit of Klenow DNA polymerase I in 20mM Tris-HC1 pH 8.0, 75 mM KC1, 5 mM MgC12, 1 mM dithiothreitol (DTT) and 100$\mu$M of the four deoxynucleoside triphosphates for 30 minutes at 37 C, spun through a Sephadex G-50 superfine column and precipitated with ethanol.

The two deoxy-oligonucleotide sequencing primers 5' dG-T-A-A-A-A-C-G-A-C-G-G-C-C-A-G-T-G 3' ((-) or master primer) and 5' dA-A-C-A-G-C-T-A-T-G-A-C-C-A-T 3' ((+) or reverse primer) were synthesized by, and obtained from, Dr J. van Boom and coworkers (Department of Organic Chemistry, Leiden University). The master (-) primer is complementary to the codogenic strand, whereas the reverse (+) primer is complementary to the non-codogenic strand of the lacZ gene (see Figure 2).

The sources of the other biochemicals used have been given previously (Konings, 1980; Peeters et al.,1983,1985)

(c) Transformation and calculation of the number of phages and transducing particles

E. coli was made competent and transformed by the method of Mandel & Higa (1970) as described by Maniatis et al (1982).

If required, the cells were infected by IKe or Ff 10 minutes prior to CaCl$_2$ treatment.

Transformed cells were spread on 2YT agar plates (Miller et al., 1972) containing 100 µg/ml of ampicillin and 0.004% of X-gal when appropriate.

The number of infective phages and ampicillin resistance transducing particles were determined by centrifugation of 100 µl of culture for 5 min in an Eppendorf centrifuge. Subsequently 50 µl of the supernatant was transferred to another tube and incubated for 2 min at 65 C to kill the remaining bacteria. 10 µl aliquots of appropriate dilutions were then added to 100 µl of exponentionally growing E. coli cells and incubated at 37°C for 10 min. The mixtures were spread on 2YT agar plates containing ampicillin (100 µg/ml) and incubated overnight at 37°C. The number of ampicillin-resistant colonies formed was used to calculate the number of transducing particles present in the original culture. Similarly the number of infective phages was determined by plating serial dilutions on an appropriate indicator strain.

(d) Isolation of single-stranded DNA
and sequence analysis

JM83/N3/F' harboring plasmid pKUN9 was grown at 37°C until the culture had reached a density of 5x10$^8$ cells/ml. Subsequently the cells were infected with either bacteriophage IKe-9 or IR1 at a multiplicity of infection of 20. After incubation overnight, 1.5 ml of culture was centrifuged for 5 minutes in an Eppendorf microfuge and 1 ml supernatant was transferred to a fresh tube

-14-

250 μl of 2.5 M NaCl, 20% polyethylene glycol-6000 was added, mixed and the mixture was allowed to stand at room temperature for 15 minutes. After centrifugation for 10 minutes in an Eppendorf microfuge the supernatant was removed and the precipitate was dissolved in 100 μl of TE (10 mM Tris-HCl pH 7.6, 1 mM EDTA) and extracted once at 65°C with 100 μl of TE-saturated phenol and once at room temperature with 100 μl of TE-saturated phenol/chloroform (1:1). The DNA in the aqueous layer was precipitated with 2.5 volumes of ethanol and after centrifugation the precipitate was washed once with 70% ethanol, dried and dissolved in 20 μl of TE.

DNA sequence analysis was performed by the dideoxy-method described by Sanger et al. (1977) using 5-10 μl of template DNA. In case packaging was instructed by phage IR1, the single-stranded DNA (F-strand) was sequenced with the aid of the reverse (+) sequencing primer whereas the master (-) primer was used for the sequence analysis of the DNA strand (I-strand) packaged under the instruction of IKe-9 (see Figure 2). Commercially available sequencing primers can be used as well for that matter.

## B. CONSTRUCTION OF THE PLASMID pKUN9

For the construction of the plasmid pKUN9 we chose pUC9 (Vieira & Messing, 1982) as parental plasmid because it contains several attractive features for cloning. Foreign DNA can be inserted at a variety of restriction endonuclease cleavage sites within a short polylinker DNA, present in the coding sequence of the α-peptide of β-galactosidase. This allows the direct identification of recombinant clones without the need of replica-plating on appropriate indicator plates (Messing,1983).

Plasmid pUC9 contains unique recognition sequences for each of the restriction enzymes NarI and NdeI. These sites are located close to one another between the lac-Z gene (pUC9 does not contain the complete lac-Z gene, but only the 5' fragment including promotor and operator) and the gene coding for ampicillin resistance (see Figure 1). Because the NarI-site has been succesfully used for the construction of the pEMBL-plasmids (Dente et al., 1983), we anticipated that both sites could be used for the insertion of foreign DNA without affecting the properties of plasmid pUC9.

The plasmid pIKori-2b (1) was constructed as follows. In the plasmid pUR222 (Ruether et al., 1981) the small PvuII fragment in the lacZ gene, which contains the multiple cloning sites was replaced by the corresponding fragment of the lacZ gene of phage M13mp9. The resulting plasmid, called pBP9, contains, as a result, besides the cloning sites present in pUR222, unique sites for SmaI (XmaI) and HindIII.

Subsequently, the AluI-B fragment (807n) of the intergenic region of IKe in the SmaI position of plasmid pBP9 was cloned. This plasmid was called pIKori-2. Finally a Bal-31 deletion mutant was obtained from the plasmid pIKori-2, in which the 3' end of the IKe insert is at position 6621. This plasmid pIKori-2b (1) contains both the IKe functional (+)-origin and the morphogenetic signal of this bacteriophage (see Figure 1).

The Ike-insert was removed from this plasmid by digestion with EcoRI and BamHI, treated with Klenow DNA Polymerase I to create blunt ends and subsequently cloned into the NdeI-site of pUC9 which had also been treated with Klenow DNA Polymerase I.

0209204

-16-

Because insertion of the IKe-fragment into the NdeI site of pUC9 does not result in a selectable phenotypical change, recombinant plasmids were selected by making use of the fact that the ColE1-origin of pUC9 is not functional in cells lacking DNA polymerase I (Bolivar et al., 1977). In cells in which this enzyme is absent (polA mutants) pUC9-derived recombinant plasmids cannot replicate unless another functional origin is present. The ligation mixture was therefore used to transform polA cells which had been infected with IKe to provide in trans a source for gene II protein. Ampicillin-resistant colonies were obtained indicating that the IKe (+)-origin had been succesfully cloned. The plasmid DNA of several colonies was isolated and used to transform strain JM83. After selection on plates containing ampicillin and X-gal, the plasmid DNA of several blue colonies was isolated and analyzed by restriction enzyme digestion. The results demonstrated that the IKe fragment had been cloned in both orientations. The plasmid that contained the (+)-origin of IKe in an orientation identical to that of the lac-Z gene of pUC9 was called pUCI-2e (see Figure 1).

The functional (+)-origin plus the morphogenetic signal of the Ff phage f1 is contained in an RsaI-fragment that almost completely spans the phage intergenic region (Hill & Petersen, 1982). The same fragment is also present in the f1 DNA sequence present in the pEMBL-plasmids (Dente et al., 1983). After digestion of plasmid pEMBl8(-) with RsaI the 514 base-pair fragment containing the (+)-origin and the morphogenetic signal of f1 was isolated. Subsequently this fragment was ligated into the NarI site of pUCI-2e which previously had been treated with Klenow DNA polymerase I to create blunt ends. After transformation of

IR1-infected polA cells, ampicillin-resistant colonies were obtained and their plasmid DNA was isolated and used to transform strain JM83. Analysis of the plasmid DNA of several blue ampicillin-resistant colonies by restriction enzyme digestion indicated that all plasmids had an identical structure and contained the f1 (+)-origin in an orientation opposite to that of IKe and the lacZ gene. The construction and structure of this plasmid, called pKUN9, is schematically shown in Figure 1.

C. Construction of the pKUN19

The parental plasmid was pUC19 (Yanisch-Perron et al., 1985) After isolation the plasmid was cleaved by means of the restriction enzymes EcoRI and HindIII, which cleave the polylinker sequence in the lacZ gene, and subsequently with the restriction enzymeHaeII , which cleaves the pUC19 plasmid at various sites, whereby it is inactivated (see Figure 2). After digestion the DNA fragments were added to the fragments obtained by digestion of the plasmid pKUN9 with the restriction enzymes EcoRI, HindIII PstI. These enzymes cleave the polylinker.sequence of pKUN9, whereby it is inactivated. To the resulting mixture ligase was added. The recombinant DNA molecules formed were then transformed to E. coli JM83 or JM101 (Yanisch-Perron et al., 1985). After transformation the bacteria were plated on 2YT agar containing ampicillin and X-gal as selection marker. The plasmids from a number of blue colonies obtained were tested for their restriction enzyme cleavage patterns, which showed conclusively that the polylinker sequence originating from the pUC9 had been replaced by that of the plasmid pUC19. Owing to this exchange

-18-

the vector plasmid pKUN19 has now obtained four unique restriction enzyme cleavage sites relative to the plasmid pKUN9 (for the enzymes SphI, XbaI, KpnI en SstI), so that its utility as a cloning vector has been further enhanced.

### D. Selective and efficient packaging of the DNA strands of pKUN9

To obtain evidence that both under the instruction of IKe-9 and of IR1, single-stranded DNA of pKUN9 is efficiently packaged into ampicillin resistance transducing particles, JM83(N3/F') cells harboring pKUN9 were grown to a density of $5 \times 10^8$ cells/ml and subsequently infected with either IKe-9 or IR1 (multiplicity of infection of 20). After 12 hours of infection the number of infective phages and transducing particles was established. It was found that the number of transducing particles present in the supernatant of both the IKe-9 and the IR1 infected cells is virtually identical to the number of infective phage particles. Similar observations were made after isolation of the single-stranded DNA from the culture supernatants and subsequent fractionation on agarose gels. In each DNA preparation two species of single-stranded DNA molecules were present in approximately equal amounts, one corresponding to phage DNA and the other to plasmid DNA. From these data it was therefore concluded that under the instruction of both IKe-9 and IR1, single-stranded DNA of pKUN9 is efficiently packaged into phage-like particles.

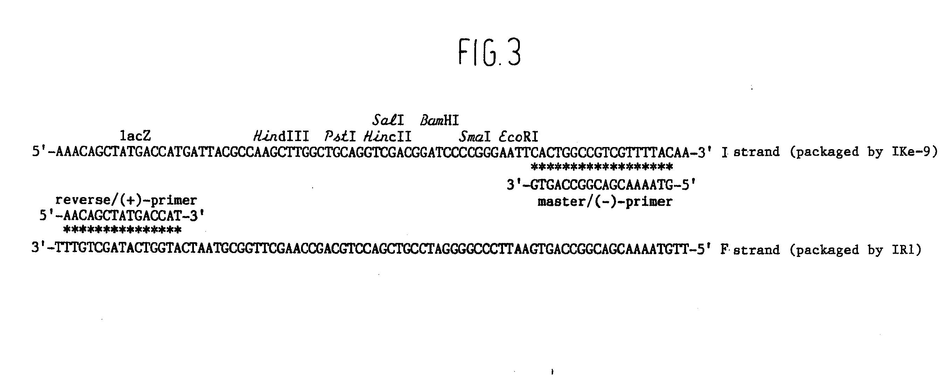

The fact that the gene II protein of IKe is unable to replicate DNA molecules containing only the (+)-origin of Ff, and vice versa, suggests that in IKe-9 infected cells the I-strand and in IR1-infected cells the F-strand of plasmid pKUN9 is selectively packaged into transducing phage-like particles. To provide evidence that this assumption is indeed correct, the nucleotide sequence of the lacZ gene present in the single-stranded DNA isolated from the culture supernatant of IKe-9 and IR1 infected pKUN9 harboring cells was established with the aid of the master and reverse sequencing primes (see: (A) (a)). Because these primers are complementary to only one strand of the lacZ gene (which is present in both the I and the F strand of pKUN9 but not in the phage DNA), sequence analysis using these primers will determine whether only one or both strands of pKUN9 are present in the single-stranded DNA preparations. As shown in Figure 3, the nucleotide sequence of the lacZ gene present in the single-stranded DNA isolated from transducing particles produced by IKe-9 infected cells can only be established using the master primer whereas for the analysis of the nucleotide sequence of the lacZ gene present in the single-stranded DNA isolated from transducing particles produced by IR1 infected cells, the reverse primer must be used. Similar conclusions were reached after dot-blot hybridization of the single-stranded DNA preparations using the same primers as probe. The master primer only hybridized to single-stranded DNA packaged by IKe-9 whereas the reverse primer only hybridized to IR1 packaged DNA.

These observations prove conclusively that after infection of pKUN9 harboring cells with IKe-9, the I strand, and after infection with IR1, the complementary (F strand) are efficiently and selectively packaged into phage-like transducing particles. Moreover, the results demonstrate that the single-stranded DNA isolated from these particles can easily by used for nucleotide sequence analysis.

Literature:

Bolivar, F., Rodriguez, R.L., Greene, P.J., Betlatch, M.C., Heynecker, H.L. & Boyer, H.W. (1977) Gene 2, 95-113.

Ciliberto, G., Raugei, G. Costanzo, F., Dente, L. & Cortese, R. (1983) Cell 32, 725-733.

Deininger, P.L. (1983) Anal. Biochem. 135, 247-263.

Denhardt, D.T., Dressler, D. & Ray, D.S. eds (1978) The Single-Stranded DNA Phages, Cold Spring Harbor Laboratory, Cold Spring Harbor, New York.

Dennison, S. & Baumberg, S. (1975), Mol. gen. Genet. 138, 321-331.

Dente, L., Cesareni, G. & Cortese, R. (1983) Nucl. Acids Res. 11, 1645-1655.

Dotto, G.P. & Zinder, N.D. (1983) Virology 130, 252-256 Dotto, G.P. Horiuchi, K. & Zinder, N.D. (1981) Virology 114, 463-473.

Gellert et al. (1977), Proc. Natl. Acad. Sci. U.S.A. 74, 4772-4776.

Guo, L-H., & Wu, R. (1982) Nucl. Acids Res. 10, 2065-2084 Heidecker, G. & Messing, J. (1983) Nucl. Acids Res. 11, 4891-4906.

Hill, D.F. & Petersen, G.B. (1982) J. Virol. 44, 32-46.

Hu, N. & Messing, J. (1982) Gene 17, 271-277

Kathoon, H. Iyer, R.V. & Iyer, V. (1972) Virology 48, 145-155.

Konings, R.N.H. (1980) Meth. Enzymol. 65, 795-811.

Mandel, M. & Higa, A. (1970), J. Mol. Biol. 53, 154-161.

Maniatis, T., Fritsch, E.F. Sambrook, J. (1982) Molecular Cloning, Cold Spring Harbor, New York.

Messing, J. (1979) Recombinant Technical Bulletin

2, 43-48.

Messing, J., Crea, R. & Seeburg, P.H. (1981) Nucl. Acids Res. 9, 309-321.

Messing, J. (1983) Meth. Enzymol. 101, 20-78.

Meyer, T.F., Geider, K., Kurz, C. & Schaller, H. (1979) Nature 278, 365-367.

Meyer, T.F. & Geider, K. (1982) Nature 296, 828-832.

Miller, J.H. (1972) Experiments in Molecular Genetics, Cold Spring Harbor, New York.

Old, R.W. & Primrose, S.B. (1982) Principles of Gene Manipulation, 2nd edition, Blackwell Scientific Publications, London.

Peeters, B.P.H., Konings, R.N.H. & Schoenmakers, J.G.G. (1983), J. Mol. Biol. 169, 197-215.

Peeters, B.P.H., Peters, R.M., Schoenmakers, J.G.G. & Konings, R.N.H. (1985) J. Mol. Biol. 181, 27-39.

Ruether, U., Koenen, M., Otto, K. & Mueller-Hill, B. (1981) Nucl. Acids Res. 9, 4087-4098.

Sanger, F., Nicklen, S. & Coulson, A.R. (1977) Proc. Natl. Acad. Sci. USA 74, 5463-5467.

Sanger, F., Coulson, A.R., Barrel, B.G., Smith, A.J.H. & Roe, B.A. (1980) J. Mol. Biol. 143, 161-178.

Slocombe, P., Easton, A., Boseley, P. & Burke, D.C. (1982) Proc.Natl.Acad. Sci. USA, 79, 5455-5459.

Vieira, J. & Messing, J.M. (1982) Gene 19, 269-276.

Wallace, R.B., Johnson, M.J., Suggs, S.V., Miyoshi, K., Bhatt, R. & Itakura, K. (1981) Gene 16, 21-26.

Yanisch-Perron, C., Vieira, J. & Messing, J. (1985), Gene 33, 103-119.

Zoller, M.J. & Smith, M. (1982), Nucl. Acids Res. 10, 6487-6500.

-22-

CLAIMS

1.     A vector plasmid built up from a starting vector incorporating, in opposite orientation relative to each other:
a) the replication origin + morphogenetic signal, or an active mutant thereof, of a filamentous bacteriophage of E. coli bacteria having F pili; and
b) The replication origin + morphogenetic signal, or an active mutant thereof, of a filamentous bacteriophage of E. coli bacteria having N pili.

2.     A vector plasmid as claimed in claim 1 which contains:
a) the replication origin + morphogenetic signal, or an active mutant thereof, of the filamentous bacteriophage Ff (M13, fd and f1) of a mutant thereof; and
b) the replication origin + morphogenetic signal, or an active mutant thereof, of the filamentous bacteriophage IKe of a mutant thereof.

3.     A vector plasmid as claimed in claim 1 or 2, based on a pUC plasmid as the starting vector.

4.     A vector plasmid as claimed in claim 3, based on the plasmid pUC9 as the starting vector.

5.     A vector plasmid as claimed in claim 4, in which (a) has been inserted in the unique recognition sequence for the restriction enzyme NarI and (b) has been inserted in the unique recognition sequence for the restriction enzyme NdeI, or the other way round.

6.     A vector plasmid pKUN9 having the structure shown in figure 1.

7. A vector plasmid pKUN19 having the structure illustrated in Fig. 2.

8. A vector plasmid as claimed in any of claims 1-7, into which a piece of double-stranded DNA has been inserted at a suitable location.

9. Microorganisms, in particular E. coli bacteria, containing a vector plasmid as claimed in any of claims 1-8.

10. E. coli bacteria sensitive to infection by F-specific and/or N-specific filamentous bacteriophages and containing a vector plasmid as claimed in any of claims 1-8.

11. A method of selectively packaging one of the strands or each of the two strands of a piece of double-stranded DNA in single-stranded DNA containing phage-like particles, in which E. coli bacteria susceptible to infection by F-specific filamentous bacteriophages and containing a vector plasmid according to claim 8 are infected with F-specific filamentous bacteriophages, and after incubation the phage-like transducing particles are isolated, and/or E. coli bacteria susceptible to infection by N-specific filamentous bacteriophages and containing a vector plasmid according to claim 8 are infected with N-specific filamentous bacteriophages, and after incubation the phage-like transducing particles are isolated, or a portion of a population of E. coli bacteria sensitive to both F-specific and N-specific filamentous bacteriophages and containing a vector plasmid according to claim 8 are infected with F-specific filamentous bacteriophages and another part of said population with N-specific filamentous bacteriophages, and after incubation the phage-like transducing particles formed are isolated.

-24-

12.   A method of determining the nucleotide sequence of a piece of double-stranded DNA, which comprises determining, in a manner known per se, the nucleotide sequence of each of the two strands of the piece of DNA concerned after obtaining these strands separately by the method according to claim 11.

13.   The use of a vector plasmid according to claim 8 for cloning DNA or messenger RNA, DNA sequencing, site directed mutagenesis, S1 mapping, expressing cloned DNA, and the preparation of a single-stranded hybridization probe.

14.   A method constructing a vector plasmid according to claim 1, which comprises cloning (a) or (b) in a restriction enzyme recognition sequence of a parental vector, selecting transformants, cloning (b) or (a) therein in another restriction enzyme recognition sequence of the parental vector, and selecting transformants.

15.   A method of constructing a vector plasmid according to claim 5, which comprises cloning (a) or (b) in the unique recognition sequence for the restriction enzyme NarI or that for the restriction enzyme NdeI of pUC9, selecting transformants by means of a polA mutant of E. coli infected with F-specific or N-specific filamentous bacteriophages, subsequently cloning (b) or (a) in one of the resulting recombinant plasmids in the unique recognition sequence not yet used, and selecting transformants by means of a polA mutant of E. coli infected with N-specific or F-specific filamentous bacteriophages.

16.   A method of constructing the vector plasmid pKUN9, which comprises treating the plasmid pUC9 with the restriction enzyme NdeI and Klenow DNA polymerase I to form a blunt-ended, linear, double-stranded DNA, ligating said linear DNA with a blunt-ended, linear, double-stranded DNA produced by treating the plasmid pIKori 2b (1) with the restriction enzymes EcoRI and BamHI, isolating the fragment containing the (+)origin + morphogenetic signal of IKe, and treating it with Klenow DNA polymerase I, selecting transformants containing the replication origin + morphogenetic signal of IKe inserted in the NdeI position of pUC9 by transformation of a polA mutant of E. coli infected with IKe or a mutant thereof, and selection of ampicillin-resistant colonies, isolating the recombinant plasmid containing the (+)-origin of IKe in the same orientation as the lacZ gene of pUC9, treating this plasmid with the restriction enzyme NarI and Klenow DNA polymerase I to form blunt-ended, linear, double-stranded DNA, ligating said linear DNA with a blunt-ended,linear, double-stranded DNA produced by treating the plasmid pEMBL8 with the restriction enzyme RsaI, isolating the fragment containing the (+)origin + morphogenetic signal of fl, and treating it with Klenow DNA polymerase I, and selecting transformants containing the replication origin + morphogenetic signal of fl inserted in the NarI position of pUC9 by the transformation of a polA mutant of E. coli infected with Ff or a mutant thereof and the selection of ampicillin-resistant colonies.

17. A method of constructing the vector plasmid pKUN19, which comprises treating the plasmid pUC19 with the restriction enzymes EcoRI, HindIII and subsequently with HaeII, isolating the products of the digestion and adding these to the fragments produced by digestion of the plasmid according to claim 6 with the restriction enzymes EcoRI, HindIII and PstI, adding ligase to this mixture, and transforming the DNA molecules formed to suitable E. coli and selecting these on agar plates containing ampicillin
and X-gal.

FIG.1

# FIG.2

pUC-19 ACGCCAAGCTTGCATGCCTGCAGGTCGACTCTAGAGGATCCCCGGGTACCGAGCTCGAATTCACTG

Hind III  Sph I  Pst I  Sal I / Acc I / Hinc II  Xba I  BamH I  Sma I / Xma I  Kpn I  Sst I  EcoR I

pUC 19

pKUN9 (4000)

Polylinker sequence

# FIG.3

```
                                SalI BamHI
       lacZ                 HindIII PstI HincII      SmaI EcoRI
5'-AAACAGCTATGACCATGATTACGCCAAGCTTGGCTGCAGGTCGACGGATCCCCGGGAATTCACTGGCCGTCGTTTTACAA-3' I strand (packaged by IKe-9)
                                                          *****************
                                                   3'-GTGACCGGCAGCAAAATG-5'
   reverse/(+)-primer                                 master/(-)-primer
 5'-AACAGCTATGACCAT-3'
   **************

3'-TTTGTCGATACTGGTACTAATGCGGTTCGAACCGACGTCCAGCTGCCTAGGGGCCCTTAAGTGACCGGCAGCAAAATGTT-5' F strand (packaged by IR1)
```

0209204

European Patent
Office

**EUROPEAN SEARCH REPORT**

Application number

EP 86 20 1252

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 141 362 (G. TAMURA) * Example 1; page 14, lines 34-36 * | 1-14 | C 12 N 15/00<br>C 12 N 1/20 //<br>( C 12 N 1/20<br>C 12 R 1:19 ) |
| | --- | | |
| A | GENE, vol. 33, no. 3, March 1985, pages 341-349, Elsevier Science Publishers, NL; K.GEIDER et al.: "A plasmid cloning system utilizing replication and packaging functions of the filamentous bacteriophage fd" * Whole document * | 1-14 | |
| | --- | | |
| A | FR-A-2 516 094 (THE WELLCOME FOUNDATION LTD.) * Whole document * | 1-14 | |
| | ----- | | **TECHNICAL FIELDS SEARCHED (Int. Cl.4)** |
| | | | C 12 N<br>C 12 P |

The present search report has been drawn up for all claims

| Place of search THE HAGUE | Date of completion of the search 09-10-1986 | Examiner CUPIDO M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82